# FASCICULE DE BREVET EUROPEEN

(11) **EP 3 288 551 B1**
(45) Date de publication et mention de la délivrance du brevet: **31.07.2019**
(21) Numéro de dépôt: 16722319.7
(22) Date de dépôt: 28.04.2016
(51) Int. Cl.: A61K 31/202, A61K 31/355, A61K 31/375, A61K 33/30, A61K 36/185, A61P 27/02

(54) **COMPOSITION CONTENANT DE LA NORBIXINE POUR LA PROTECTION DES CELLULES DE L'EPITHELIUM PIGMENTAIRE RETINIEN**
ZUSAMMENSETZUNGEN ENTHALTEND NORBIXIN ZUM SCHUTZ VON RETINALEN PIGMENTEPITHELZELLEN
COMPOSITION CONTAINING NORBIXIN FOR THE PROTECTION OF RETINAL PIGMENT EPITHELIAL CELLS

(30) Priorité: 30.04.2015 FR 1553957
(43) Date de publication de la demande: 07.03.2018
(73) Titulaire: Biophytis, 75001 Paris (FR); Sorbonne Université, 75006 Paris (FR)
(72) Inventeur: LAFONT, René, 75016 Paris (FR); VEILLET, Stanislas, 91600 Savigny-sur-orge (FR); SAHEL, José-Alain, 75013 Paris (FR); FONTAINE, Valérie, 75013 Paris (FR); ELENA, Pierre-Paul, 06200 Nice (FR)
(74) Mandataire: Ipside
(86) Numéro de dépôt international: PCT/FR2016/051001
(87) Numéro de publication internationale: WO 2016/174360

(56) Documents cités:
- WO-A1-2005/110375
- WO-A2-01/85183
- FR-A1- 2 947 173
- FR-A1- 2 975 008
- JP-A- 2010 285 364

## Description

### Domaine de l'invention

La présente invention vise le domaine des traitements des cellules de l'épithélium pigmentaire rétinien (EPR).

Plus particulièrement, la présente invention vise l'utilisation d'une composition pour la protection des cellules de l'épithélium pigmentaire rétinien (EPR), notamment pour le traitement de la dégénérescence maculaire liée à l'âge (DMLA) ou encore de la maladie de Stargardt et la rétinite pigmentaire chez les mammifères.

L'invention a pour but d'améliorer la vision des individus atteint de ces maladies ou au moins de stabiliser l'évolution de la maladie. La présente invention concerne une composition comportant plus de 90% en poids de Norbixine obtenue par purification à partir d'un extrait de graines de Bixa orellana, pour son utilisation pour la photoprotection des cellules de l'épithélium pigmentaire rétinien (EPR) chez le mammifère par administration de ladite composition audit mammifère, par jour, en quantité comprise entre 0,48 mg/kg de poids corporel et 48 mg/kg de poids corporel.

### État de la technique

La dégénérescence maculaire liée à l'âge (DMLA) est une cause de cécité irréversible chez les populations de personnes âgées, en particulier en Europe et en Amérique du Nord. La DMLA affecte la partie centrale de la rétine, appelée macula, entraînant une grave déficience visuelle et la perte irréversible de la vision centrale.

La fonction maculaire est à l'origine de la vision centrale et de l'acuité visuelle dont la haute résolution est liée à sa forte concentration en photorécepteurs à cônes. Le stade précoce de la DMLA est marqué par des dépôts appelés Drüsen, qui n'affectent la vision que de façon marginale. Les phases ultérieures comprennent deux formes de DMLA, l'atrophie géographique (forme sèche) ou exsudative (forme humide ou néovasculaire), la première étant beaucoup plus fréquente que la seconde. Les dernières étapes de ces deux formes conduisent à la destruction de la rétine neurosensorielle maculaire, mais l'évolution de la DMLA sèche est généralement lente, alors que la DMLA humide peut conduire à la cécité complète en quelques semaines.

Le vieillissement est l'accumulation progressive avec le temps de changements qui sont associés à (ou responsables de) une susceptibilité croissante de la maladie. Dans la rétine, un certain nombre de maladies dégénératives, y compris le glaucome, la rétinite pigmentaire et la DMLA, peuvent survenir à la suite du vieillissement. La rétinite pigmentaire regroupe un ensemble hétérogène de dégénérescences rétiniennes génétiques, impliquant les photorécepteurs et l'EPR, et conduisant à une perte de vision nocturne, puis tardivement de la vision centrale. Bien que les mécanismes spécifiques impliqués dans l'initiation de différents types de maladies liées au vieillissement rétinien diffèrent, on pense que le stress oxydatif et l'inflammation qui en résultent sont des éléments importants qui contribuent à la pathogenèse.

Les théories de l'étiologie de la DMLA incluent des modifications hydrodynamiques dans la membrane de Bruch causées par une accumulation progressive de matériel extracellulaire contenant des lipides, et la sénescence de l'EPR, dont l'activité est indispensable à la survie des photorécepteurs. Les cellules de l'EPR ont plusieurs fonctions différentes dans les yeux: elles établissent la barrière hémato-rétinienne par leurs jonctions serrées, et sont ainsi responsables du statut immuno-privilégié de la partie intérieure de l'ampoule de l'oeil ; elles maintiennent en vie les photorécepteurs en leur apportant des nutriments et participent au cycle visuel. La compréhension actuelle est qu'une déficience de la fonction des cellules de l'EPR est à l'origine du développement de la DMLA. Le vieillissement provoque un dysfonctionnement des cellules de l'EPR et une insuffisance de leur métabolisme, ainsi que de leur activité phagocytaire. Une digestion incomplète des segments externes des photorécepteurs peut conduire à la formation de Drüsen en réduisant la diffusion à travers la membrane de Bruch, ce qui provoque dans un premier temps une déformation de la rétine et des images perçues.

Avec l'âge, l'EPR stocke une quantité croissante de lipofuscines. Celles-ci sont composées de lipides et de protéines, qui proviennent des phagolysosomes, des lysosomes et des photorécepteurs. Les lipofuscines contiennent également de la N-rétinyl-N-rétinylidène éthanolamine (A2E), qui est formée par la condensation de deux molécules de rétinaldéhyde avec de l'éthanolamine.

Le vieillissement s'accompagne d'une accumulation accrue d'A2E dans la rétine (Bhosale et al., 2009). Sous l'action de la lumière bleue et en présence d'oxygène, l'A2E génère des espèces réactives qui provoquent des dommages aux protéines, aux lipides et à l'ADN, et donc un stress oxydatif important dans les cellules vieillissantes de l'EPR (Sparrow & Cai, 2001). Ces dommages perturbent l'activité lysosomiale des cellules de l'EPR et provoquent une accumulation de déchets, ce qui finit par engendrer, de place en place, la mort des cellules de l'EPR qui est suivie par celle des photorécepteurs auxquels elles étaient associées.

Aucun médicament n'existe sur le marché pour le traitement de la DMLA sèche, alors que des médicaments par injection intravitréenne d'anticorps anti-VEGF (Vascular Endothelial Growth Factor) sont commercialisés permettant de bloquer partiellement la formation de néovaisseaux et offrant ainsi une alternative de traitement de la DMLA humide. Des compléments alimentaires ont été formulés avec des composés antioxydants génériques, à savoir des minéraux et des vitamines aux propriétés antioxydantes, par exemple le zinc, les vitamines A, C, E, avec une efficacité thérapeutique réelle mais limitée. La formule AREDS nutraceutique 1 (en anglais « Age-Related Eye Disease Study », AREDS 2001) est considérée comme la norme de soins aux États-Unis pour le traitement de la DMLA sèche, réduisant le risque de DMLA avancée de 25 % et la perte de vision de 19 % sur cinq ans.

De nombreux produits proposent une base de formulation commune : Zinc et vitamines C et E, à laquelle sont ajoutés divers ingrédients : lutéine, resvératrol, acides gras Oméga 3, sans toutefois disposer de données d'efficacité convaincantes sur ces ingrédients additionnels, ni sur les catégories de patients susceptibles de répondre favorablement à ces différentes molécules (Elliot & Williams, 2012). En particulier l'art antérieur connait la demande internationale WO 2005110375 qui concerne un complément alimentaire destiné à limiter les limiter ou prévenir la perte d'acuité visuelle des suites d'une maladie oculaires.

Les caroténoïdes (des molécules exclusivement apportées par l'alimentation), ont été plus particulièrement étudiés, car certains d'entre eux (lutéine, zéaxanthine = des xanthophylles) sont naturellement présents dans la macula (Subczynski et al., 2010), et l'on sait que ces composés possèdent un fort pouvoir antioxydant. C'est donc logiquement que ces composés ont été testés (seuls ou en combinaison) dans la formule AREDS, mais les résultats obtenus ont été limités, la supplémentation ne s'avérant efficace que pour un sous-ensemble de patients carencés en ces composés (Pinazo-Durán et al., 2014). Ces molécules sont efficaces *in vitro* pour protéger des cellules de l'EPR (Human D407) contre les effets toxiques du peroxyde d'hydrogène (Pintea et al., 2011).

La demande de brevet japonais JP 2010285364 met en avant un mélange composé de crocétine et d'un autre caroténoïde pouvant être une xanthophylle ou un autre diapocaroténoïde, i.e. la bixine ou la norbixine. Ce mélange, du fait de ses propriétés anti-oxydantes, est proposé pour soulager ou prévenir des maladies dans lequel un phénomène oxydatif est impliqué.

D'autres xanthophylles ont également fait l'objet d'études par supplémentation orale, seules ou en combinaison avec la lutéine et/ou la zéaxanthine (ex. astaxanthine - Parisi et al., 2008). Récemment, des diapocaroténoïdes (= caroténoïdes tronqués aux deux extrémités - IUPAC chemical nomenclature) ont été testés *in vitro* et *in vivo,* en particulier la crocétine (= 8,8'-diapocarotene-8,8'-dioate) et ses glycosides (les crocines). Les crocines présentent un effet photoprotecteur *in vitro* sur des cultures primaires de photorécepteurs de bovins ou de primates (Laabich et al., 2006), et la crocétine protège les cellules neuroganglionnaires contre un stress oxydatif (Yamauchi et al., 2011). Le safran (une épice riche en crocines/crocétine) administré per os s'est avéré actif *in vivo* sur la qualité de la rétine (Maccarone et al., 2008; Falsini et al., 2010; Boisti et al., 2014). Toutefois, le safran contenant d'autres molécules susceptibles d'être actives sur la rétine, comme d'autres caroténoïdes ainsi que du safranal formé en même temps que la crocétine (Verma & Middha, 2010 ; Fernández-Sánchez et al., 2012), il est difficile de conclure quant à l'effet de la seule crocétine.

Des expérimentations ont également été réalisées avec un autre apocaroténoïde, la bixine (= methylhydrogen 6,6'-diapocarotene-6,6'-dioate) ou certains de ses dérivés, *in vitro* sur des cellules neuroganglionnaires et *in vivo* par injections intravitréennes pour contrecarrer les effets d'un stress du réticulum endoplasmique (Tsuruma et al., 2012). Les tests ainsi réalisés évaluent le plus souvent une activité antioxydante et donc protectrice des composés vis-à-vis de divers types cellulaires de la rétine soumis à la présence d'un agent oxydant (ex. le peroxyde d'hydrogène), et ils ne se situent donc pas directement dans le contexte de la DMLA.

Un extrait de graines d'Urucum (*Bixaorellana*) précédemment développé (Bixilia®) a montré un effet photoprotecteur sur la peau humaine exposée aux UV (FR 2947173, Veillet et al., 2009) et sur des cellules d'EPR soumises à un stress photooxydant (Fontaine et al., 2011). L'extrait Bixilia® est un extrait naturel d'Urucum qui a été enrichi en Bixine. Bixilia® contient d'autres composés photoprotecteurs de nature phénolique, dont la présence pourrait expliquer l'activité photoprotectrice supérieure de l'extrait brut par rapport à la Bixine seule. Dans le brevet FR 11 54172 (Fontaine et al., 2011 , publié comme FR 2 975 008), l'effet protecteur des cellules d'EPR de certains des composés de l'extrait Bixilia® est analysé. Les résultats des tests utilisant la Bixine ou Norbixine aux concentrations 0,1 micromolaire (µM), 1 µM et 10 µM n'ont pas d'activité photoprotectrice et laissent même penser que plus la concentration en Bixine ou Norbixine est grande, moins les cellules d'EPR survivent et donc moins l'effet photoprotecteur est grand. On remarque, entre autres, que des substances telles que la cyanidine et l'acide ellagique à des concentrations de 10 µM et 20 µM ont un effet photoprotecteur avantageux sur les cellules d'EPR.

Une étude approfondie a conduit à identifier les molécules actives présentes dans l'extrait Bixilia® et à préciser leur mécanisme d'action, puis à démontrer leur efficacité *in vivo* chez la souris et le rat. Cette étude a donné naissance à la présente invention. L'invention prévoit ainsi de trouver un traitement alternatif à ceux déjà existants pour la protection des cellules d'EPR.

### Objet de l'invention

Les inventeurs ont découvert que la Norbixine, en particulier sa forme 9'-*cis,* permettait de diminuer fortement la mort cellulaire provoquée par une illumination avec des rayonnements bleus des cellules de l'EPR prétraitées avec de la N-rétinyl-N-rétinylidène éthanolamine (A2E).

Selon un premier aspect, la présente invention vise une composition comportant plus de 90% en poids de Norbixine obtenue par purification à partir d'un extrait de graines de *Bixa orellana,* pour son utilisation pour la photoprotection des cellules de l'épithélium pigmentaire rétinien (EPR) chez le mammifère par administration de ladite composition audit mammifère, par jour, en quantité comprise entre 0,48 mg/kg de poids corporel et 48 mg/kg de poids corporel.

Dans le cadre de l'invention, on entend par « extrait de graines de *Bixa orellana* » un extrait préparé à partir de la partie externe des graines, c'est-à-dire de la substance cireuse recouvrant les graines de *Bixa orellana.* Cette substance cireuse est connue pour être riche en bixine et en d'autres caroténoïdes mineurs, ainsi que pour son utilisation comme colorant alimentaire.

La Norbixine, biodisponible chez les mammifères après administration orale, est bien mieux absorbée que la Bixine et se retrouve dans l'oeil, en particulier dans la rétine.

Dans des modes de réalisation de l'invention, la composition comporte plus de 90 % en poids de Norbixine.

Dans des modes de réalisation particuliers de l'invention, la composition comporte plus de 95 % en poids de Norbixine.

Dans des modes de réalisation particuliers de l'invention, la composition comporte plus de 90 % en poids de Norbixine sous sa forme 9'-*cis* de formule (I) :

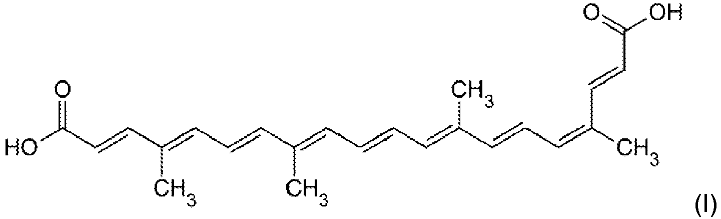

Dans des modes de réalisation particuliers, la composition comporte au moins un élément choisi parmi le zinc, la vitamine C et la vitamine E.

Dans des modes de réalisation particuliers, la composition peut être utilisée sous forme d'un complément alimentaire ou d'un médicament.

Par complément alimentaire on entend produit renfermant ladite composition ayant pour objet de compléter l'alimentation en apportant des nutriments bénéfiques pour la santé selon la définition donnée par la directive européenne 2002/46/EC. Par exemple, un complément alimentaire peut être une gélule ou comprimé à avaler ou une poudre ou petite ampoule à mélanger à un aliment et présentant des effets bénéfiques sur les cellules d'EPR.

Par médicament on entend produit contenant une dose précise dudit composé ou dudit extrait selon la définition donnée par la directive européenne 65/65/CE à savoir toute substance ou composition présentée comme possédant des propriétés curatives ou préventives à l'égard des maladies humaines ou animales. Par exemple, le médicament contenant le composé aux doses thérapeutiques peut-être administré par voie orale sous forme de gélule ou comprimé ou injecté par voie intra-vitréenne ou tout autre voie permettant de conférer des effets bénéfiques sur la rétine.

Dans des modes de réalisation particuliers, la composition comprend un support acceptable pour être ingéré, injecté dans l'oeil, injecté par voie systémique ou injecté dans le sang.

Dans les modes de réalisation, la composition est administrée au mammifère, par jour, en quantité comprise entre 0,48 mg/kg de poids corporel et 48 mg/kg de poids corporel, de préférence comprise entre 0,6 mg/kg de poids corporel et 20 mg/kg de poids corporel.

Selon d'autres modes de réalisation particuliers de l'invention, la composition est destinée à prévenir les dommages à la rétine susceptibles d'être causés par l'exposition aux rayonnements bleus. Par rayonnement bleus, on entend les rayonnements correspondant à la bande bleue du spectre de la lumière visible, de longueur d'onde comprise entre 435 nm et 490 nm.

Dans des modes de réalisation particuliers de l'invention, la composition est destinée au traitement de la dégénérescence maculaire liée à l'âge (DMLA) chez le mammifère.

Dans d'autres modes de réalisation particuliers, la composition est destinée à traiter la maladie de Stargardt et/ou la rétinite pigmentaire chez le mammifère. La maladie de Stargardt, ou syndrome de Stargardt, est une pathologie héréditaire, associant une baisse d'acuité visuelle bilatérale à une atrophie de la macula, qui reproduit, à un âge précoce, les symptômes de la forme sèche de la DMLA.

### Brève description des figures

La Figure 1 illustre le pourcentage de cellules d'EPR survivantes en présence de N-rétinyl-N-rétinylidène éthanolamine (A2E) et de l'extrait Bixilia® ou de Bixine (20 µM) ou de Norbixine (20 µM) après avoir été soumises à une illumination.
La Figure 2 illustre l'activité photoprotectrice des extraits successifs des graines d'Urucum (C = cyclohexane; D = dichlorométhane; M = méthanol) sur les cellules de l'EPR mises en présence d'A2E et soumises à une illumination.
La Figure 3a illustre les concentrations plasmatiques après ingestion de Bixine (à gauche) ou de Norbixine (à droite) chez la souris C57BI/6.
La Figure 3b illustre l'analyse pharmacocinétique de la Norbixine chez la souris C57BI/6.
La Figure 4 illustre l'analyse par HPLC-MS/MS de la Norbixine dans les yeux de souris double KO (ABCA4^{-/-}, RDH8^{-/-}) après injection intrapéritonéale (10 mg/kg). (3 : la Norbixine injectée, 1 et 2 : mono-glucuronides de ce composé).
La Figure 5 illustre la cinétique de l'accumulation de l'A2E dans l'oeil de souris double KO (ABCA4^{-/-}, RDH8^{-/-}) en fonction de l'âge, en comparaison avec des souris normales.
La Figure 6 illustre les électrorétinogrammes (Ondes A à gauche et Ondes B à droite) de souris double KO (ABCA4^{-/-}, RDH8^{-/-}) ayant reçu des injections intravitréennes unilatérales de Norbixine (afin d'obtenir une concentration finale dans le vitré de 130 µM), placées pendant 24 h à l'obscurité puis exposées à la lumière bleue (4000 lux, 1 h). Les électrorétinogrammes sont réalisés 7 jours après l'illumination.
La Figure 7 illustre le nombre de couches de noyaux de photorécepteurs en fonction de la distance à partir du nerf optique dans les yeux de souris traitées comme dans la figure 6.
La Figure 8A illustre le protocole expérimental de la création du modèle « rat blue light ».
La Figure 8B illustre les résultats des électrorétinogrammes des rats auxquels il a été injecté de la norbixine (100 mg/kg, 4 injections par rat d'une solution à 50 mM dans du NaCl 9 ‰, 4 rats/série) en utilisant le PBN (phényl-N-tert-butylnitrone, 50 mg/kg, solution à 20 mg/mL dans du NaCl 9 ‰) comme témoin positif. Les électrorétinogrammes sont réalisés 7 jours après le traitement.
La Figure 9A illustre le nombre de couches de noyaux de photorécepteurs en fonction de la distance à partir du nerf optique dans les yeux de rats après injections intrapéritonéales d'alpha-phényl-N-tert-butylnitrone (PBN) ou de Norbixine et illumination avec une lumière bleue. Les analyses histologiques sont réalisées 7 jours après le traitement.
La Figure 9B illustre l'aire sous chaque courbe de la Figure 9A.
La Figure 10 illustre la quantité d'A2E accumulée dans l'oeil de souris double KO (ABCA4^{-/-}, RDH8^{-/-}) ayant ingéré ou non de la nourriture complémentée en norbixine pendant 3 mois.
La Figure 11 illustre les électrorétinogrammes de souris double KO (ABCA4^{-/-}, RDH8^{-/-}) ayant été nourries ou non avec de la nourriture contenant 0,3 mg/g de norbixine pure pendant 3 mois.
La Figure 12 illustre la relation entre l'amplitude de l'électrorétinogramme (onde A) et la quantité d'A2E accumulée dans les yeux des souris double KO (ABCA4^{-/-}, RDH8^{-/-}).
La Figure 13 illustre les résultats de l'analyse par HPLC en phase inverse de la norbixine purifiée à partir de l'extrait de *Bixa orellana* (l'identification des isomères a été réalisée selon Scotter et al., 1998 et Polar-Cabrera et al., 2010).

### Description détaillée d'exemples de réalisation de l'invention

A la différence de la plupart des études précédemment publiées, les modèles utilisés dans la présente invention (à la fois *in vitro* et *in vivo*) mettent en avant le rôle de la N-rétinyl-N-rétinylidène éthanolamine (A2E) et de sa phototoxicité, et sont à ce titre, plus proches de la pathologie humaine. Les tests utilisés *in vitro* s'apparentent dans leur principe à ceux utilisés avec d'autres substances naturelles sur une lignée de cellules d'EPR humain (cellules ARPE-19 - Young et al., 2005).

### Protocoles et Résultats

### 1- Préparation de la Bixine et de la Norbixine

La Bixine pure à 95% en poids est préparée à partir d'un produit commercial (Annatto B) provenant d'une extraction organique des graines d'Urucum et d'une concentration en Bixine supérieure à 85% en poids. La purification est réalisée par recristallisations successives.

La Norbixine pure à 95% en poids est obtenue après hydrolyse alcaline de la Bixine purifiée (5% KOH, 60°C, 3 heures). La solution obtenue est acidifiée par de l'acide chlorhydrique concentré et la Norbixine est récupérée par centrifugation. Le culot est lavé deux fois par de l'eau pour éliminer les sels, et le culot final est lyophilisé.

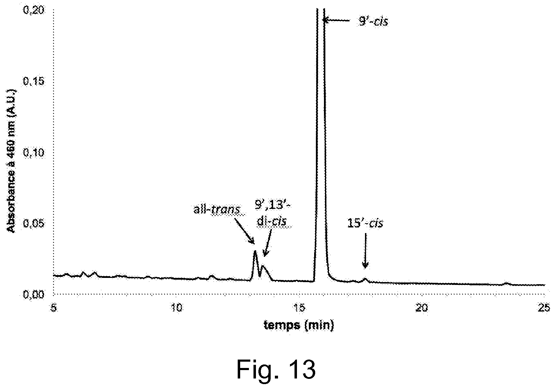

La pureté des composés est appréciée par spectrophotométrie UV-Vis et HPLC en phase inverse : les composés contiennent essentiellement les isomères 9'-*cis* (concentration supérieure à 90% en poids, figure 13).

### 2- Tests in vitro

Un test *in vitro* destiné à étudier l'effet photoprotecteur de diverses substances naturelles sur les cellules de l'EPR mises en présence d'A2E a été utilisé. L'effet photoprotecteur des molécules est évalué dans un modèle cellulaire de phototoxicité induite par traitement par A2E suivi d'une illumination en lumière bleue. Par rayonnements bleus on entend les rayonnements correspondant à la bande bleue du spectre de la lumière visible, soit de longueur d'onde comprise entre 435 et 490 nm.

Ce modèle utilise des cultures primaires d'EPR de porc adulte. La survie cellulaire est quantifiée grâce à un test de viabilité cellulaire. On ajoute à -48h les composés à tester (en solution 5 mM dans le DMSO) pour obtenir des concentrations finales de 1 à 20 µM) puis à -19h de l'A2E (concentration finale 30 µM) et on irradie les cellules (temps 0h). On mesure 24h après la survie des cellules. L'acquisition des images ainsi que leur traitement sont réalisés à l'aide d'un microscope à fluorescence piloté par le logiciel Metamorph et d'un programme de quantification dédié. Les expériences sont réalisées sur des microplaques de 96 puits en quadruplicate et l'expérience est reproduite au minimum quatre fois. Les résultats sont exprimés sous forme d'un ratio représentant le nombre de cellules vivantes dans les puits traités par les molécules à tester, divisé par le nombre de cellules vivantes dans les puits contrôles (traités par le milieu de dilution sans A2E).

Ce test a permis précédemment de mettre en évidence la très bonne activité photoprotectrice d'un extrait éthanolique de graines d'Annatto (Bixilia® - voir Fontaine et al., 2011). Dans le travail précédent, si l'activité de l'extrait d'Annatto avait été démontrée, la nature de la (ou des) substance(s) photoprotectrice(s) n'avait pas été identifiée, et le composant principal de cet extrait (la bixine) s'était avéré inefficace aux concentrations de 0,1 µM, 1 µM et 10 µM. Un travail complémentaire a donc été entrepris afin d'identifier le (ou les) composé(s) actif(s).

### a. La Bixine (non-revendiquée) est responsable d'une grande partie de l'activité photoprotectrice de Bixilia®

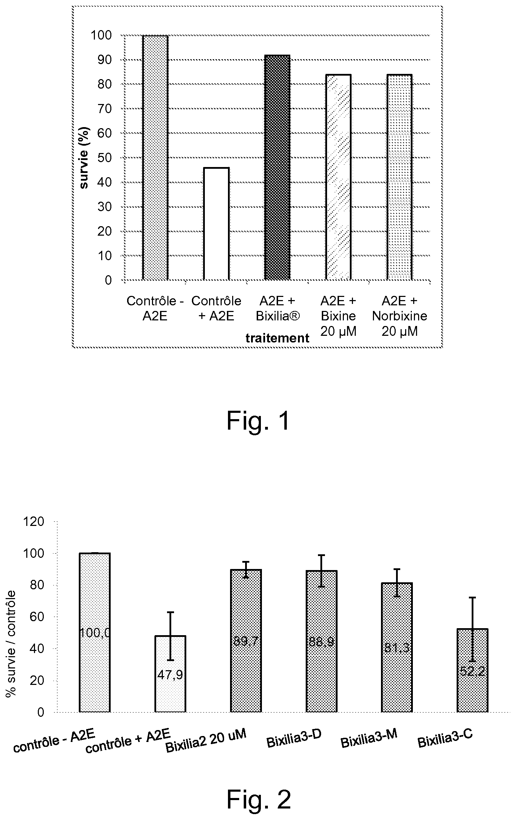

La Figure 1 montre que la Bixine et la Norbixine (20 µM) protègent efficacement les cellules de l'EPR contre la phototoxicité induite en présence d'A2E par rapport au contrôle avec A2E. Un extrait brut de graines d'Urucum dilué pour apporter 20 µM de bixine possède une activité photoprotectrice élevée. L'utilisation de bixine très pure et à la concentration de 20 µM a permis de montrer que ce composant possédait en fait une activité photoprotectrice importante (Figure 1) et que celle-ci expliquait une part importante de l'activité de l'extrait Bixilia® dilué de façon à apporter la même quantité de bixine. Une activité comparable a également été trouvée pour la Norbixine, qui représente le métabolite circulant majeur de la Bixine (Lévy et al., 1997). Ces résultats sont en accord avec l'activité photoprotectrice de ces mêmes composés, précédemment démontrée pour la photoprotection de la peau humaine (Veillet et al., 2009).

### b. Bixilia® contient d'autres composés photoprotecteurs (non-revendiqués)

Bixilia® contient d'autres composés photoprotecteurs de nature phénolique, dont la présence pourrait expliquer l'activité supérieure de l'extrait brut par rapport à la bixine seule (pour une même concentration en bixine). Une extraction séquentielle de l'enveloppe des graines d'Urucum a été réalisée avec successivement du cyclohexane, du dichlorométhane et du méthanol (1 L de chaque / 100 g de graines). Après extraction au cyclohexane on obtient une fraction avec une concentration en Bixine de 0,65 µM, après extraction au dichlorométhane on obtient une fraction qui possède une concentration en Bixine de 1485 µM, et après extraction au méthanol une fraction qui possède une concentration en Bixine de 45 µM.

On reproduit alors le test *in vitro* précédent.

Selon la Figure 2 la fraction dichlorométhane, qui contient 97 % de la Bixine, est très active, mais on note également que l'extrait méthanolique riche en composés phénoliques possède une activité significative (C = cyclohexane; D = dichlorométhane; M = méthanol).

### 3- Biodisponibilité de la Bixine et de la Norbixine

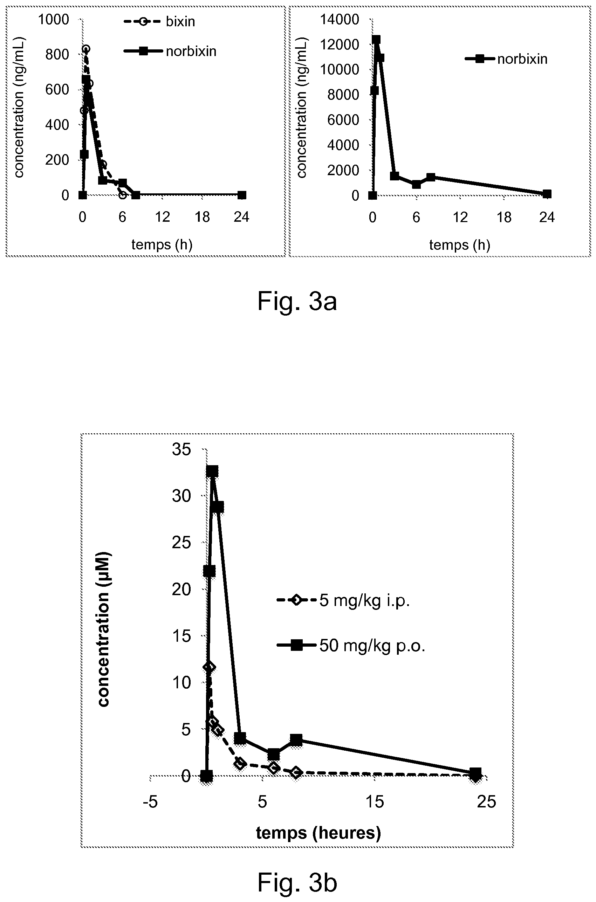

Des études de la biodisponibilité de la Bixine et de la Norbixine ont été réalisées chez des souris C57BI/6. Les composés ont été administrés par voie orale (50 mg/kg). Des prélèvements sanguins ont été effectués après 0,25, 0,5, 1, 3, 6, 8 et 24 h et analysés par HPLC-DAD (UV 460 nm)-MS/MS. Le tableau 1 et la Figure 3a dévoilent que la Bixine ingérée est rapidement transformée en Norbixine et que les deux composés circulent à des concentrations comparables, on ne les détecte plus après 8 heures. On note par ailleurs que la Norbixine ingérée est nettement plus biodisponible que la Bixine.

**Tableau 1 :**

| Ingestion de bixine (50 mg/kg) | | | Ingestion de norbixine (50 mg/kg) | | |
|---|---|---|---|---|---|
| Tₘₐₓ (h) | Cₘₐₓ (ng/mL) | AUC 0-24h (ng.h/mL) | Tₘₐₓ (h) | Cₘₐₓ (ng/mL) | AUC (ng.h/mL) |
| Bixine 0,5 | 833 | 1666 | Norbixine 0,5 | 12400 | 41609 |
| Norbixine 0,5 | 649 | 1343 | | | |

La comparaison des analyses plasmatiques (figure 3b) après injection intrapéritonéale (5 mg/kg) et administration orale (50 mg/kg) montre que la biodisponibilité de la Norbixine est de 55%.

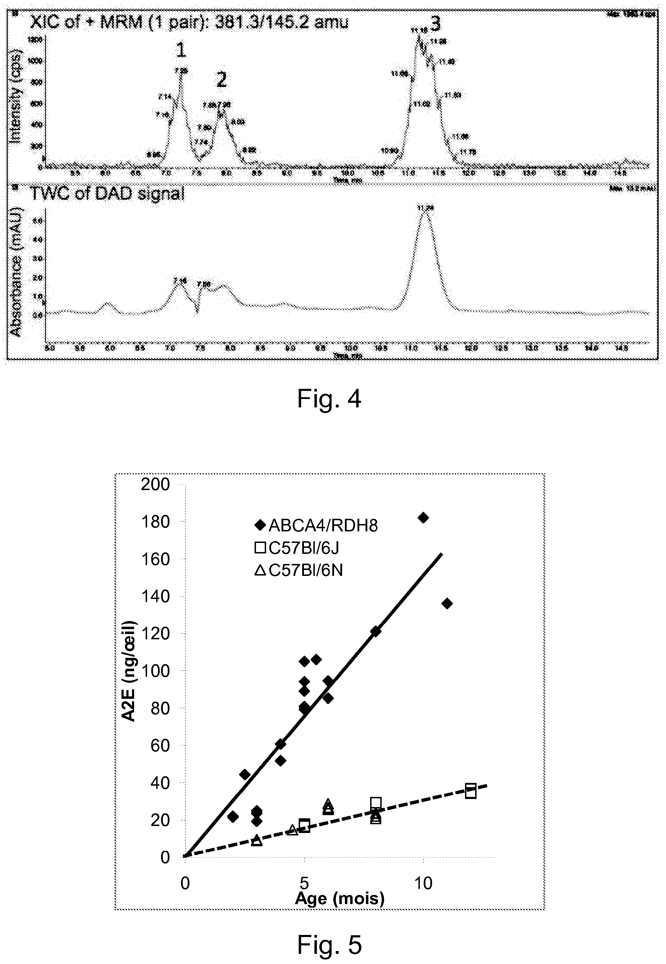

La présence de Norbixine dans les yeux a été recherchée chez des souris double KO (ABCA4^{-/-}, RDH8^{-/-}) 3 heures après injection intrapéritonéale de Norbixine (10 mg/kg). Les yeux de 6 animaux ont été disséqués et les échantillons ont été extraits avec de l'acétonitrile, poolés puis analysés par HPLC-MS/MS (Figure 4), ce qui a permis de détecter spécifiquement la présence de la Norbixine dans l'EPR et la rétine (Tableau 2).

**Tableau 2 :**

| Echantillon | Norbixine (ng/organe) |
|---|---|
| EPR | 5,15 |
| Rétine | 2,40 |
| Cristallin | < LOQ |
| Humeur vitrée | < LOQ |
| Total | 7,55 |

Selon la Figure 4, on note que, dans le plasma, mais aussi dans les yeux, la Norbixine est également présente sous forme conjuguée : le composé initial donne en effet deux mono-glucuronides qui sont élués avant le composé original et présentent une fragmentation similaire, sans doute due à la décomposition des glucuronides dans la source du spectromètre de masse. La glucuronidation avait également été décrite dans le cas de la crocétine (Asai et al., 2005).

On peut également observer une isomérisation *cis-trans* de la Norbixine, dont l'importance varie selon la durée des expérimentations. C'est un phénomène classique chez les composés (poly)-insaturés, qui correspond à des isomérisations *cis-trans* d'une ou plusieurs doubles liaisons et a été observé chez l'homme dans le cas de la norbixine par Lévy et al. (1997). Le composé ici utilisé est purifié à partir de composés commerciaux (Annatto B) ; il contient très majoritairement la forme 9-*cis* et de très petites quantités de la forme all*-trans* et d'autres formes *cis* ou di-*cis* (figure 13).

### 4- Activité photoprotectrice par injection intravitréenne chez la souris

Un modèle de souris génétiquement modifiée développé par Maeda et al. (2008) a été utilisé pour tester l'activité photoprotectrice de la Norbixine. Dans ce modèle de souris, deux gènes impliqués dans le cycle du pigment visuel (ABCA4 et RDH8) sont inactivés, ce qui se traduit par une accumulation précoce d'A2E dans les yeux (Figure 5). Ce modèle animal s'apparente de ce fait à la pathologie humaine, avec bien sûr ses limites, liées aux différences de l'organisation des yeux entre rongeurs et primates.

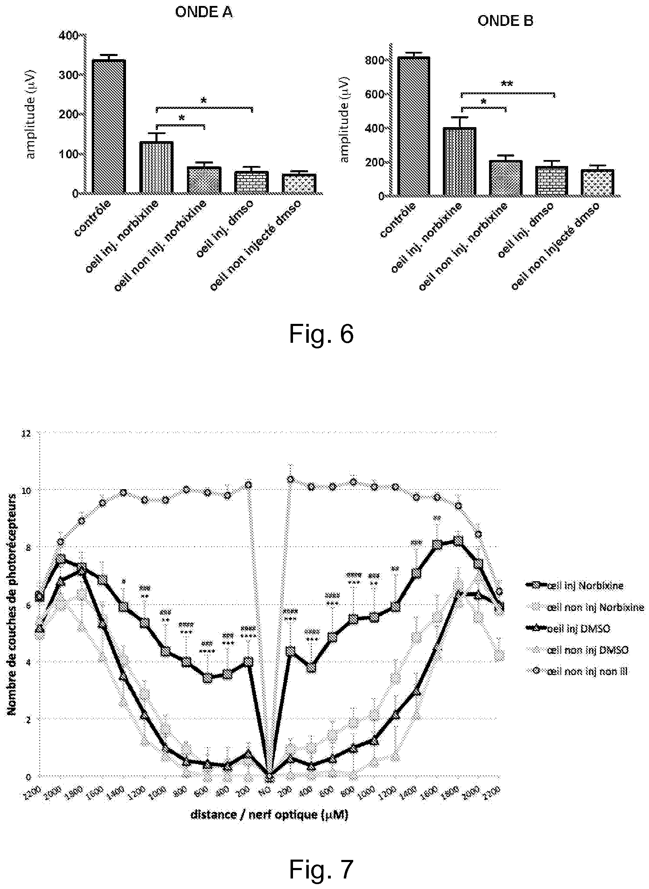

Des souris âgées de 7 semaines ont donc été utilisées pour réaliser des injections intravitréennes unilatérales de Norbixine (afin d'obtenir une concentration finale dans le vitré de 130 µM). Après 24 h à l'obscurité, les souris ont été soumises à une exposition à la lumière bleue (4000 lux, 1 h). Les électrorétinogrammes réalisés 7 jours plus tard ont montré un effet protecteur de la Norbixine, dont la présence a permis de maintenir une activité électrique significative comme exposé par la Figure 6.

Une étude histologique de l'épaisseur de la couche de noyaux externes met en évidence l'effet protecteur de la Norbixine sur les photorécepteurs (figure 7). Il faut noter que la Norbixine a pratiquement été éliminée 24 h après l'injection intravitréenne et n'est donc que très faiblement présente dans les yeux au moment de l'illumination.

### 5- Activité photoprotectrice par injection systémique (intrapéritonéale) chez le rat

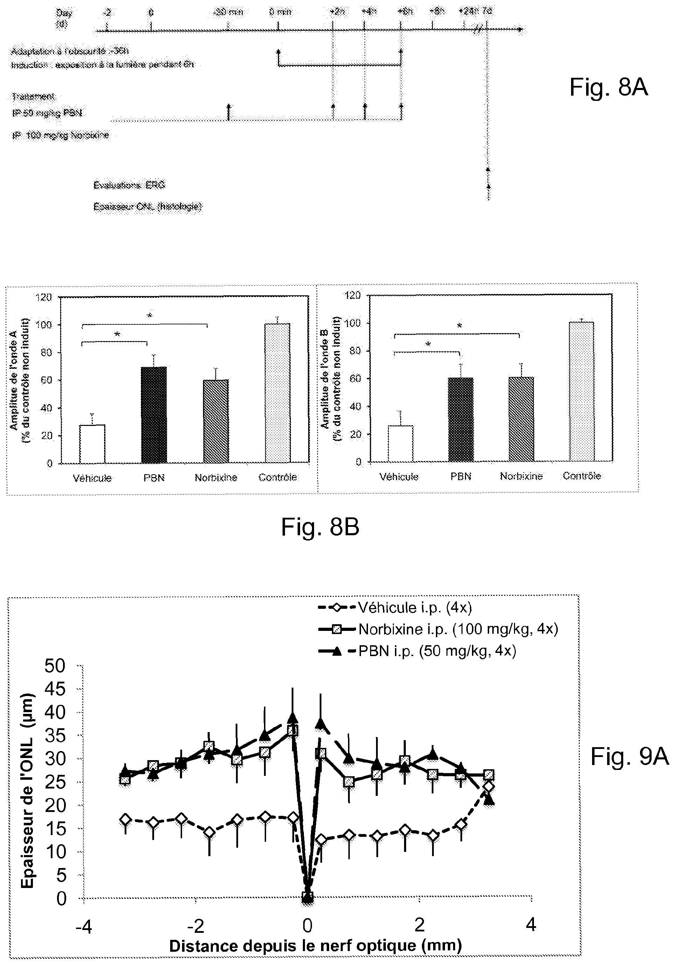

Le modèle "rat blue light" consiste à soumettre les animaux à une lumière bleue intense pendant 6 heures afin de provoquer des dommages oculaires que l'on apprécie 7 jours plus tard par la réalisation d'électrorétinogrammes puis par des analyses histologiques. On utilise un composé antioxydant, le PBN (phényl-N-tert-butylnitrone) comme témoin positif (Ranchon et al., 2001; Tomita et al., 2005). Les composés dont on cherche à déterminer l'activité photoprotectrice sont injectés (par voie intrapéritonéale) avant et pendant la phase d'illumination. Celle-ci est réalisée avec des tubes néon Philips blue (4,2 mW/cm²) pendant 6 heures. Le protocole expérimental est présenté en Figure 8A.

Trois séries d'expérimentations ont été réalisées avec la Norbixine (100 mg/kg, Quatre injections par rat d'une solution à 50 mM dans du NaCl 9 ‰, 4 rats/série) en utilisant le PBN (phényl-N-tert-butylnitrone, 50 mg/kg, solution à 20 mg/mL dans du NaCl 9 ‰) comme témoin positif. L'analyse des électrorétinogrammes (ondes A et B) est présentée en Figure 8B.

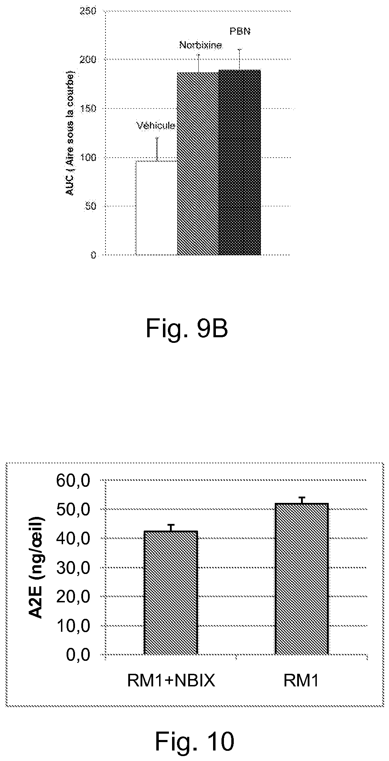

Ce test a permis de mettre en évidence une efficacité significative de la Norbixine, qui est proche de celle du PBN. Les données histologiques correspondantes (figure 9A et 9B) confirment l'action photoprotectrice de la Norbixine sur la survie des photorécepteurs.

### 6- Activité photoprotectrice par administration orale chronique chez la souris

Un aliment contenant 0,3 mg/g de Norbixine pure a été préparé et donné à des souris double KO (ABCA4^{-/-}, RDH8^{-/-}) pendant une période de 3 mois.

Les animaux ayant reçu la nourriture supplémentée avec de la Norbixine montrent une réduction de l'accumulation d'A2E dans les yeux (figure 10) : la différence entre les deux groupes est très significative (p = 0.0109).

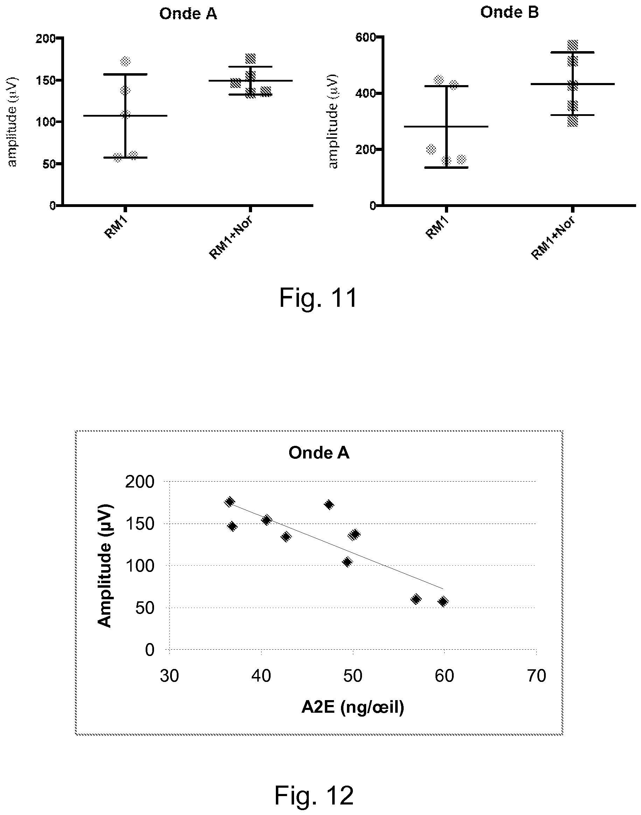

La nourriture complémentée en Norbixine présente également un effet positif sur l'amplitude de l'électrorétinogramme (ERG) (figure 11).

Ces analyses ont également montré qu'il existe une relation inverse entre la quantité d'A2E accumulée dans les yeux et l'amplitude de l'ERG (figure 12), ce qui confirme le rôle de l'accumulation d'A2E dans le développement de la pathologie (Wu et al., 2014), et l'intérêt de molécules dont l'administration réduit l'accumulation de l'A2E dans les yeux.

Cependant, on n'observe pas d'accumulation significative de Norbixine dans les yeux au cours de ce traitement chronique, ce qui amène à conclure que, à la différence des xanthophylles, cette molécule serait dégradée. La non-accumulation de cette substance active peut être considérée comme un avantage, car l'accumulation de certains caroténoïdes (ex. canthaxanthine) est susceptible de conduire à la formation de dépôts au sein des cellules de l'EPR (Goralczyk et al., 1997). Elle est également indicatrice d'une action modificatrice de l'activité des cellules de l'EPR plutôt que d'un rôle de filtre ou d'antioxydant, comme postulé pour la lutéine et la zéaxanthine. Ce résultat rejoint celui noté lors des injections intravitréennes (à savoir la disparition de la Norbixine au moment de l'illumination).

La dose journalière permettant de ralentir significativement la dégénérescence rétinienne chez la souris après administration orale est de 48 mg/kg de poids corporel. La transposition à l'homme conduit à proposer une dose journalière active de 4,8 mg/kg. On sait par ailleurs que la dose journalière admissible ou DJA (en anglais : *Acceptable Daily Intake* ou ADI) de la Norbixine est au maximum de 0,6 mg/kg/jour de poids corporel (JECFA/67/FC). Cette valeur a été établie sur la base d'une dose sans effet ou DES (en anglais : *No Observable Adverse Effect Level* ou NOAEL) chez le rat de 69 mg/kg/jour de poids corporel, équivalent à une dose journalière sans effet chez l'homme de 11 mg/kg, sachant qu'aucune toxicité n'a été observée jusqu'à 20 mg/kg/jour (Hagiwara et al., 2003). La posologie proposée est comprise entre 0,48 mg/kg/jour et 48 mg/kg/jour, idéalement entre 0,6 mg/kg/jour et 20 mg/kg/jour.

### Références

AREDS Report No. 8. 2001. A randomized, placebo-controlled, clinical trial of high-dose supplementation with vitamins C and E, beta carotene, and zinc for age-related maculardegeneration and vision loss. Arch Ophthalmol, 119: 1417-1436.
Asai A, Nakano T, Takahashi M, Nagao A. 2005. Orally administered crocetin and crocins are absorbed into blood plasma as crocetin and its glucuronide conjugates in mice. J Agric Food Chem, 53: 7302-7306.
Bhosale P, Serban B, Bernstein PS. 2009. Retinal carotenoids can attenuate formation of A2E in the retinal pigment epithelium. Arch BiochemBiophys, 483: 175-181.
Bisti S, Maccarone R, Falsini B. 2014. Saffron and retina: neuroprotection and pharmaco-kinetics. Visual Neurosci, 1-7. doi:10.1017/S0952523814000108.
Chábera P, Fuciman M, Hribek P, Polivka T. 2009. Effect of carotenoid structure on excited-state dynamics of carbonyl carotenoids.PhysChemChemPhys, 11: 8795-8803.
Elliott JG, Williams NS. 2012. Nutrients in the battle against age-related eye diseases. American Optometric Association. doi:10.1016/j.optm.2011.11.006
Falsini B, Piccardi M, Minnella A, Savastano C, Capoluongo E, Fadda A, Balestratti E, Maccarone R, Bisti S. 2010. Influence of saffron supplementation on retinal flicker sensitivity in early age-related macular degeneration. InvestOphthalmol Vis Sci, 51: 6118-6124.
Fernândez-Sânchez L, Lax P, Esquiva G, Martin-Nieto J, Pinilla I, Cuenca N. 2012. Safranal, a saffron constituent, attenuatesretinaldegeneration in P23H rats. PLoS ONE, 7(8): e43074.
Fontaine V, Lafont R, Sahel JA, Veillet S. 2011. Utilisation de composés et composition pour le traitement de la dégénérescence maculaire liée à l'âge (DMLA). Demande FR 25506 (déposée le 14 mai 2011).
Goralczyk R, Buser S, Bausch J, Bee W, Zühlke U, Barker FM. 1997. Occurrence of biréfringent retinal inclusions in cynomolgus monkeys after high doses of canthaxanthin. Invest Ophthalmol Vis Sci, 38: 741-752.
Hagiwara A, Imai N, Ichihara T, Sano M, Tamano S, Aoki H, Yasuhara K, Koda T, Nakamura M, Shirai T. 2003. A thirteen-week oral toxicity study of annatto extract (norbixin), a natural food color extracted from the seed of annatto (Bixaorellana L.), in Sprague-Dawley rats. Food ChemToxicol, 41: 1157-1164.
Laabich A, Vissvesvaran GP, Lieu KL, Murata K, McGinn TE, Manmoto CC, Sinclair JR, Karliga I, Leung DW, Fawzi A, Kubota R. 2006. Protective effect of crocin against blue light- and white light-mediated photoreceptor cell death in bovine and primate retinal primary cell culture. Invest Ophthalmol Vis Sci, 47: 3156-3163.
Lévy LW, Regalado E, Navarette S, Watkins RH. 1997. Bixin and norbixin in human plasma: Détermination and study of the absorption of a single dose of annatto food color. Analyst, 122: 977-980.
Liu X, Osawa T. 2007. Cisastaxanthin and especially 9-cisastaxanthin exhibits a higher antioxidant activity in vitro compared to the all-trans isomer. BiochemBiophys Res Comm, 357: 187-193.
Maccarone R, Di Marco S, Bisli S. 2008. Saffron supplementation maintains morphology and function after exposure to damaging light in mammalian retina. Invest Ophthalmol Vis Sci,49: 1254-1261.
Maeda T, Maeda A, Golczak M, Palczewski K. 2008. Retinopathy in mice induced by disrupted all-trans-retinal clearance. J BiolChem, 283: 26684-26693.
Maeda T, Maeda A, Matosky M, Okano K, Roos S, Tang J, Palczewski K. 2009. Evaluation of potential therapies for a mouse model of human age-related macular degeneration caused by delayed all-trans-retinal clearance.Invest Ophthalmol Vis Sci, 50: 4917-1925.
Melendez-Martinez AJ, Stinco CM, Liu C, Wang XD. 2013. A simple HPLC method for the comprehensive analysis of cis/trans (Z/E) geometrical isomers of carotenoids for nutritional studies. Food Chem, 138: 1341-1350.
Montenegro MA, De O Rios A, Mercadante AZ, Nazareno MA, Borsarelli CD. 2004. Model studies on the photosensitized isomerization of bixin. J Agric Food Chem, 52: 367-373.
Parisi V, Tedeschi M, Gallinaro G, Varano M, Saviano S, Piermarocchi S. 2008. Carotenoids and antioxidants in age-related maculopathy Italian study: multifocal electroretinogram modifications after 1 year. Ophthalmology, 115(2): 324-333.
Phan-Thi H, Waché Y. Isomerization and increase in the antioxidant properties of lycopene from Momordicacochinchinensis (gac) by moderate heat treatment with UV-Vis spectra as a marker. Food Chem, 156: 58-63.
Pinazo-Durân MD, Gómez-Ulla F, Arias L, Araiz J, Casaroli-Marano R, Gallego-Pinazo R, García-Medina JJ, López-Gálvez MA, Manzanaq L, Salas A, Zapara M, Diaz-Llopis M, García-Layana A. 2014. Do nutritional supplements have a role in age macular degeneration prévention? J Ophthalmology, article ID 901686.
Pintea A, Rugina DO, Pop R, Bunea A, Socaciu C. 2011. Xanthophylls protect against induced oxidation in cultured human retinal pigment epithelial cells. J Food Compos Anal, 24(6): 830-836.
Rios ADO, Borsarelli CD, Mercadante AZ. 2005. Thermal dégradation kinetics of bixin in an aqueous model system. J Agric Food Chem, 53: 2307-2311.
Sparrow JR, Cai B. 2001. Blue light-induced apoptosis of A2E-containing RPE: involvement of caspase-3 and protection by Bcl-2. Invest Ophthalmol Vis Sci, 42: 1356-1362.
Subczynski WK, Wisniewska A, Widomska J. 2010. Location of macular pigments in the most vulnérable régions of photoreceptor outer-segment membranes.Arch BiochemBiophys, 504: 61-66.
Tsuruma K, Shimazaki H, Nakashima K, Yamauchi M,Sugitani S, Shimazawa M, linuma M, Hara H. 2012. Annatto prevents retinal degeneration induced by endoplasmic reticulum stress in vitro and in vivo.MolNutr Food Res, 56: 713-724.
Veillet S, Lafont R, Dioh W. 2009. Cosmetic composition for protection from the sun containing urucumextract.Priority Application FR2009-54354 A (25 juin 2009), Application no FR 2009-54354, WO 2010-FR51323.
Verma RS, Middha D. 2010.Analysis of saffron (Crocus sativusL.) stigma components by LC-MS-MS. Chromatographia, 71: 117-123.
Widomska J, Subczynski WK. 2014. Why has nature chosen lutein and zeaxanthin to protect the retina? J ClinExpOphthalmol, 5(1): 326, doi: 10:4172/2155-9570.1000326.
Wu L, Ueda K, Nagasaki T, Sparrow JT. 2014. Light damage in Abca4 and Rpe65rd12 mice. Invest Ophthalmol Vis Sci, 55: 1910-1918.
Yamauchi M, Tsuruma K, Imai S, Nakanishi T, Umigai N, Shimazawa M, Hara H. 2011. Crocetin prevents retinal degeneration induced by oxidative stress and endoplasmic reticulum stress via inhibition of caspase activity. Mol Cell Pharmacol, 650: 110-119.
Young JP, Zhou J, Nakanishi K, Sparrow JN. 2005. Anthocyanins protect against A2E photooxidation and membrane permeabilization in retinal pigment epithelial cells. PhotochemPhotobiol, 81: 529-536.

## Revendications

1. Composition comportant plus de 90% en poids de Norbixine obtenue par purification à partir d'un extrait de graines de *Bixa orellana,* pour son utilisation pour la photoprotection des cellules de l'épithélium pigmentaire rétinien (EPR) chez le mammifère par administration de ladite composition audit mammifère, par jour, en quantité comprise entre 0,48 mg/kg de poids corporel et 48 mg/kg de poids corporel.

2. Composition pour son utilisation selon la revendication 1, étant administrée au mammifère, par jour, en quantité comprise entre 0,6 mg/kg de poids corporel et 20 mg/kg de poids corporel.

3. Composition pour son utilisation selon l'une quelconque des revendications 1 ou 2, comportant plus de 95% en poids de Norbixine.

4. Composition pour son utilisation selon l'une quelconque des revendications 1 à 3, comportant plus de 90% en poids de Norbixine sous sa forme *9'-cis* de formule (I) :

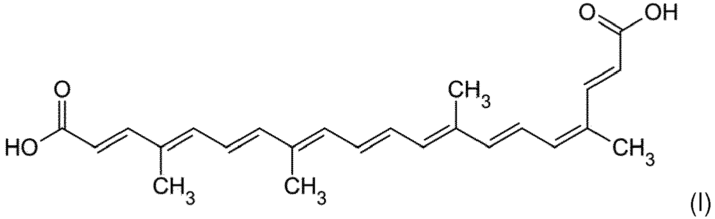

5. Composition pour son utilisation selon l'une des revendications 1 à 4, comportant au moins un élément choisi parmi le zinc, la vitamine C et la vitamine E.

6. Composition pour son utilisation selon l'une des revendications 1 à 5, se présentant sous la forme d'un complément alimentaire ou d'un médicament.

7. Composition pour son utilisation selon l'une des revendications 1 à 6, comprenant un support acceptable pour être ingéré, injecté dans l'oeil, injecté par voie systémique ou injecté dans le sang.

8. Composition pour son utilisation selon l'une des revendications 1 à 7, pour son application dans la prévention des dommages à la rétine causés par l'exposition aux rayonnements bleus correspondant à la bande bleue du spectre de la lumière visible, de longueur d'onde comprise entre 435 nm et 490 nm.

9. Composition pour son utilisation selon l'une des revendications 1 à 8, pour son application dans le traitement de la dégénérescence maculaire liée à l'âge (DMLA) chez les mammifères.

10. Composition pour son utilisation selon l'une des revendications 1 à 8, pour son application dans le traitement de la maladie de Stargardt et/ou la rétinite pigmentaire chez les mammifères.

## Patentansprüche

1. Zusammensetzung, aufweisend über 90 Gew.-% Norbixin, erhalten durch Reinigung aus einem Extrakt von Samen von *Bixa orellana,* für ihre Verwendung für die Photoprotektion der Zellen des retinalen Pigment-Epithels (RPE) beim Säuger durch Verabreichung der Zusammensetzung dem Säuger pro Tag in einer Menge, die zwischen 0,48 mg/kg Körpergewicht und 48 mg/kg Körpergewicht liegt.

2. Zusammensetzung für ihre Verwendung nach Anspruch 1, wobei sie dem Säuger pro Tag in einer Menge verabreicht wird, die zwischen 0,6 mg/kg Körpergewicht und 20 mg/kg Körpergewicht liegt.

3. Zusammensetzung für ihre Verwendung nach einem der Ansprüche 1 oder 2, aufweisend über 95 Gew.-% Norbixin.

4. Zusammensetzung für ihre Verwendung nach einem der Ansprüche 1 bis 3, aufweisend über 90 Gew.-% Norbixin in seiner Form *9'-cis* der Formel (I):

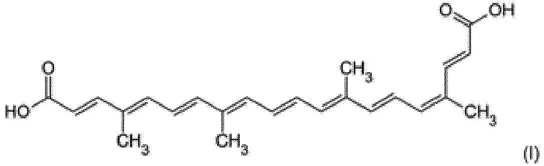

5. Zusammensetzung für ihre Verwendung nach einem der Ansprüche 1 bis 4, aufweisend mindestens ein Element, ausgewählt aus dem Zink, dem Vitamin C und dem Vitamin E.

6. Zusammensetzung für ihre Verwendung nach einem der Ansprüche 1 bis 5, vorliegend in der Form einer Nahrungsergänzung oder eines Arzneimittels.

7. Zusammensetzung für ihre Verwendung nach einem der Ansprüche 1 bis 6, umfassend einen Träger, der für Verschlucken, Injektion in das Auge, systemische Injektion oder Injektion in das Blut akzeptabel ist.

8. Zusammensetzung für ihre Verwendung nach einem der Ansprüche 1 bis 7 für ihre Anwendung bei der Vorbeugung von Retinaschädigungen, die durch Exposition gegenüber blauer Strahlung, die dem blauen Band des Spektrums des sichtbaren Lichts einer Wellenlänge entsprechen, die zwischen 435 nm und 490 nm liegt, verursacht werden.

9. Zusammensetzung für ihre Verwendung nach einem der Ansprüche 1 bis 8 für ihre Anwendung bei der Behandlung der altersbedingten Makuladegeneration (AMD) bei den Säugern.

10. Zusammensetzung für ihre Verwendung nach einem der Ansprüche 1 bis 8 für ihre Anwendung bei der Behandlung des Morbus Stargardt und/oder der Retinitis pigmentosa bei den Säugern.

## Claims

1. Composition including more than 90% by weight of Norbixin obtained by purification from a *Bixa orellana* seed extract, for use for the photoprotection of retinal pigment epithelium (RPE) cells in mammals by administering said composition to said mammal, per day, in a quantity between 0.48 mg/kg of body weight and 48 mg/kg of body weight.

2. Composition for use according to claim 1, being administered to the mammal per day, in a quantity between 0.60 mg/kg of body weight and 20 mg/kg of body weight.

3. Composition for use according to any one of claims 1 or 2, including more than 95% by weight of Norbixin.

4. Composition for use according to any one of claims 1 to 3, including more than 90% by weight of Norbixin in the *9'-cis* form thereof having formula (I):

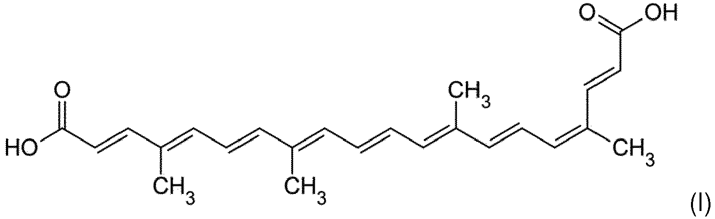

5. Composition for use according to one of claims 1 to 4, including at least one element chosen among zinc, vitamin C and vitamin E.

6. Composition for use according to one of claims 1 to 5, presented in the form of a dietary supplement or a medicinal product.

7. Composition for use according to one of claims 1 to 6, comprising an acceptable substrate for being ingested, injected into the eye, injected by the systemic route or injected into the blood.

8. Composition for use according to one of claims 1 to 7, for application in the prevention of retinal damage caused by exposure to blue light corresponding to the blue band of the visible light spectrum, of wavelength between 435 nm and 490 nm.

9. Composition for use according to one of claims 1 to 8, for application in the treatment of age-related macular degeneration (ARMD) in mammals.

10. Composition for use according to one of claims 1 to 8, for application in the treatment of Stargardt disease and/or retinitis pigmentosa in mammals.
